(19) Europäisches Patentamt
European Patent Office
Office européen des brevets

(11) EP 2 240 067 B1

(12) EUROPEAN PATENT SPECIFICATION

(45) Date of publication and mention
of the grant of the patent:
**19.08.2015 Bulletin 2015/34**

(51) Int Cl.:
***A61B 1/005*** (2006.01)

(21) Application number: **08865115.3**

(22) Date of filing: **17.12.2008**

(86) International application number:
**PCT/NL2008/050811**

(87) International publication number:
**WO 2009/082210 (02.07.2009 Gazette 2009/27)**

(54) **A BENDABLE STRUCTURE AND A METHOD FOR BENDING A STRUCTURE**

BIEGBARE STRUKTUR UND VERFAHREN ZUM BIEGEN EINER STRUKTUR

STRUCTURE PLIABLE ET PROCÉDÉ DE PLIAGE D'UNE STRUCTURE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **20.12.2007 EP 07150257**

(43) Date of publication of application:
**20.10.2010 Bulletin 2010/42**

(73) Proprietor: **Nederlandse Organisatie voor
toegepast-
natuurwetenschappelijk onderzoek TNO
2595 DA 's-Gravenhage (NL)**

(72) Inventors:
• **HEIJMANS, Jeroen Antonius Cecillia
NL-5261 EL Vught (NL)**

• **VERBAAN, Cornelis Abraham Marinus
NL-4225 PM Noordeloos (NL)**

(74) Representative: **Hatzmann, Martin et al
V.O.
Johan de Wittlaan 7
2517 JR Den Haag (NL)**

(56) References cited:
**WO-A-99/60267        WO-A-2007/034664
US-A- 4 753 223        US-A- 5 220 911
US-A- 5 679 216        US-A- 5 897 488
US-A1- 2005 154 261**

## Description

FIELD OF THE INVENTION

The invention relates to a bendable structure. In particular, the invention relates to a bendable medical instrument, like a catheter or an endoscope. The invention further relates to a method of bending a structure.

BACKGROUND OF THE INVENTION

An embodiment of a bendable structure is known from Ki-Tae Park et al "An active catheter with integrated circuit for communication and control". In the known bendable structure bending functionality is enabled by providing a tubular body of a catheter with coils fabricated from a shape memory alloy (SMA). SMA materials are known as such in the art and relate to a class of materials which may be deformed in a controlled way, for example, pursuant to heating by application of a current pulse. Details on SMA materials may be found in M. Langelaar and F. van Keulen "Modeling of a shape memory alloy active catheter".

The known bendable structure comprises a plurality of segments manufactured from SMA coils, said segments being consecutively interconnected by links. The SMA coils are pre-deformed having 3% deformation strain. When the SMA actuator is heated above its phase transition temperature by electric current and starts recovering its original shape, the active catheter bends in the direction of the heated SMA actuator. In order to implement bending of the known bendable structure a total of three SMA actuator wires are provided within a body conceived to be bent, these three actuator wires are arranged at vertices of an imaginary triangle fitted into a cross-section of the body.

It is a disadvantage of the known bendable body that bending with poor accuracy can be achieved. Next, the known bendable structure has limited bending angles. Finally, the known bendable structure cannot be adequately miniaturized due to required use of at least three SMA actuator wires.

US 5,679,216 discloses a structure according to the preamble of claim 1.

SUMMARY OF THE INVENTION

It is an object of the invention to provide a bendable structure wherein greater bending accuracy can be reached. Additionally, it is a further object of the invention to provide a bendable structure wherein greater bending angles can be enabled. It is a still further object of the invention to provide a bendable structure having substantially diminished power dissipation into surroundings upon actuation.

To this end the bendable structure according to the invention is defined by claim 1.

The invention is based on the insight that a uni-directional SMA wire, when pre-deformed, relaxes during activation (i.e. during heating). In course of relaxation a bending force induced by the relaxing SMA wire can be transferred to another side of the body across the bridge structure. Uni-directional memory material relates to a material which can revert to its original shape, which is defined a-priori. It will be appreciated that the term "uni-directional" may be substituted by a term "one-way", as both this terms are used in the art for denoting a characteristic of being capable of transiting from the martensitic phase to the austenitic phase, for example, due to heating. Upon heating, energy, stored in the pre-deformed uni-directional SMA wire is released and is not reproducible, unless the energy is stored in a second element, for example a second uni-directional SMA wire, preferably located opposite to the first SMA wire with respect to the bridge structure.

The bendable structure according to the invention is different from commonly known embodiments utilizing bi-directional SMA materials. Two stripes of bi-directional SMA materials (i.e. "two-way" SMA) may be arranged in a bendable structure, wherein each of the stripes has a different length at a different temperature, corresponding either to the martensitic or the austenitic phase. These two different lengths are reproduced each time the temperature of the SMA stripe is changed. However, energy produced by the SMA stripe during transition from one state to another is not stored in any element of the bendable structure.

There are at least two principal embodiments envisaged for the bridge structure according to an aspect of the invention. First, the body may comprise an elastic hinge, whereas the bridge structure may be formed by the elastic hinge connected to the uni-directional SMA wire. In a particular embodiment, when the elastic flexible hinge is manufactured from a SMA material, the bending force transferred from the uni-directional SMA wire can be stored in an opposite arm of the elastic hinge. This feature is based on the insight that the hinge can be implemented as a spring-like element, in particular when for a material of the flexible hinge a material having pseudo-elastic properties is selected. Advantageously, when such material exhibits austenitic properties at room temperature, it can be brought in the martensitic state by inducing deformation. When a force causing the deformation is relieved, the material will return in it original shape. In general such materials are similar to rubber when transiting from austenitic to martensitic state. It is appreciated that during transition from the martensitic to austenitic phase substantially great forces are relieved. Use of pseudo elastic material is advantageous for reasons of low bending stiffness and large allowable strain (bending angle). Secondly, choosing a uni-directional SMA for the hinge material is advantageous, due to low bending stiffness, large allowable strain, and the energy that can be stored.

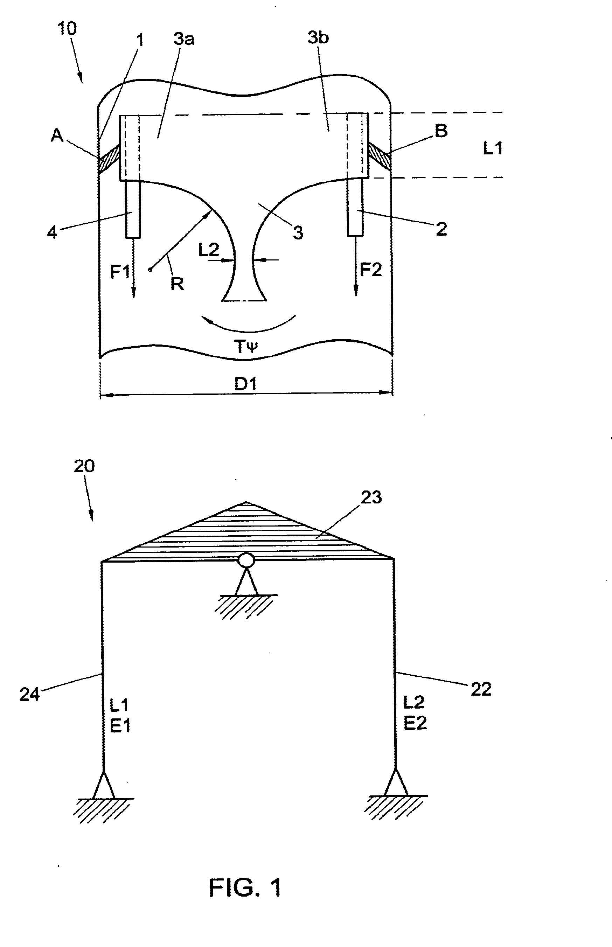

Details on operation of the bridge structure are explained with reference to Figure 1. When the opposite side of the SMA hinge is actuated, the force is transferred back to the uni-directional SMA wire. In this way bending force induced in the bendable structure is being transferred from one side of the bridge to another side of the bridge in accurate way. In addition, this embodiment uses only one actuation uni-directional SMA wire and is, therefore, preferable in situations when the bendable structure has to meet miniaturization requirements, for example for minimal invasive surgery, such as miniature endoscopes. Alternatively, a miniaturized device may be used for industrial applications, for example for purposes of engine or gear inspection.

Secondly, it is possible that the bridge structure comprises a further deformable uni-directional SMA wire joined by a rigid hinge to the pre-deformed wire.

In this case the bridge structure may represent a substantially rigid hinge connected to two deformable SMA wires acting as it arms. It will be appreciated that the term 'rigid' relates to a material of the hinge being substantially non-deformable. Preferably, the hinge material has a Young modulus of more than 1GPa at T=20°C. The hinge interconnecting the pre-deformed wire and the further wire forms a mechanical bridge for transferring a bending momentum from one side of the hinge to another thereby causing the body of the bendable structure to bend. It will be appreciated that the bridge has a pre-stored mechanical energy due to the fact that the first uni-directional SMA wire is pre-deformed. In this way, when the electrical current pulse of a suitable duration is applied to the pre-deformed first wire and the material of the wire is heated above a phase transition temperature, the SMA wire create a force on the further deformable SMA wire. In response, the second deformable uni-directional SMA wire will be elongated until equilibrium of forces across the bridge is reached. In this way the mechanical energy is transferred to the second wire and is stored there. When the second uni-directional SMA wire is being heated above its phase transition temperature by means of electrical current or otherwise, the second wire will be shortening in response and will thereby create a force on the first (now relaxed) uni-directional SMA wire. As a result the first uni-directional SMA wire will be elongated until equilibrium of forces along the bridge is reached. Now, the mechanical energy is being transferred again across the bridge and being stored in the first wire. By repeating alternating heating the first wire and the second wire the controlled bending of the bendable structure is achieved. This phenomenon is explained in more detail with reference to Figure 1.

The bendable structure according to the invention has the following advantages. First, due to provision of the bridge structure accurate bending actuation is reached due to good controllability of phase transition of the uni-directional SMA material. In addition, due to use of a uni-directional SMA material a small amount of activation energy is required to bend the bendable structure. Secondly, the activation energy is only applied for a short duration, for example a current pulse of 0.1 - 10 s duration, preferably 5s duration can be used to achieve necessary bending angle. This is advantageous with respect to the prior art where the activation pulse has to be applied continuously leading to a great energy dissipation in surroundings. This may be not acceptable for medical applications wherein heating of surrounding tissue is not allowable.

Preferably, the SMA wire is pre-deformed by elongation by 4-8% of its initial length. This means that for reaching envisaged bending radii of about 10mm for a 1mm diameter, at least 4-8% elongation of the SMA wire is advantageous. It will be appreciated that it is also possible that both SMA wires are pre-deformed for a portion of 4 - 8% range.

In an embodiment of the bendable structure according to the invention the uni-directional shape memory alloy is selected from a group of materials comprising: NiTi, CuZnAl or CuAlNi.

As has been mentioned earlier, uni-directional shape memory property is a functionality of a material to transit to its original shape from a deformed condition after it is heated. This phenomenon is based on a phase transition in the crystal structure during cooling of a rigid, high temperature state (austenitic) to a less rigid lower temperature state (martensitic) and vice versa during heating. This phase transformation is reversible and, therefore, can easily be used for actuation purposes. It will be appreciated that there is another transition possible for the shape memory alloys, namely a transition between the austenitic state and the so-called R-phase. The R-phase corresponds to rhombohedral crystal orientation. It is found that it is preferable to use the transformation between the austenitic phase and the martensitic phase in case smaller bending radii are required. For larger bending radii it is advantageous to use the transformation between the austenitic phase to the R-phase because such transition is linear as a function of temperature and has little hysteresis, which is advantageous for simplifying control of the bending.

In a further embodiment of the bendable structure according to the invention the body has a low bending stiffness, which may be implemented when for the material of the hinge a SMA material is selected, in particular a material having pseudo-elastic properties.

This is advantageous, as is this case the body demonstrates substantially no resistance to bending and substantially no backwards recoiling. This further improves bending accuracy.

In a still further embodiment of the bendable structure according to the invention, it further comprises control means for inducing a transition of the shape memory alloy from martensitic phase to austenitic phase or from austenitic phase to R-phase.

Preferably, the control means is arranged to apply a pulse of electrical current of suitable duration and/or amplitude for enabling a transition of the shape memory alloy from martensitic phase to austenitic phase or from austenitic phase to R-phase.

The control means may be advantageously arranged to use a pre-calibrated dependency between a desired bending radius of the bendable structure and the duration or amplitude of a current pulse conceived to be applied to the SMA wires for enabling such bending. This feature further improves the bending accuracy. This short term activation (0.1s - 10 s, preferably about 5s) has an advantage of reduced power dissipation on the bendable structure, which is preferable for medical applications due to strict limitation on local heating of tissue.

In a further embodiment of the bendable structure according to the invention the elastic hinge comprises a plurality of interconnected hinge elements extending in a longitudinal direction of the body.

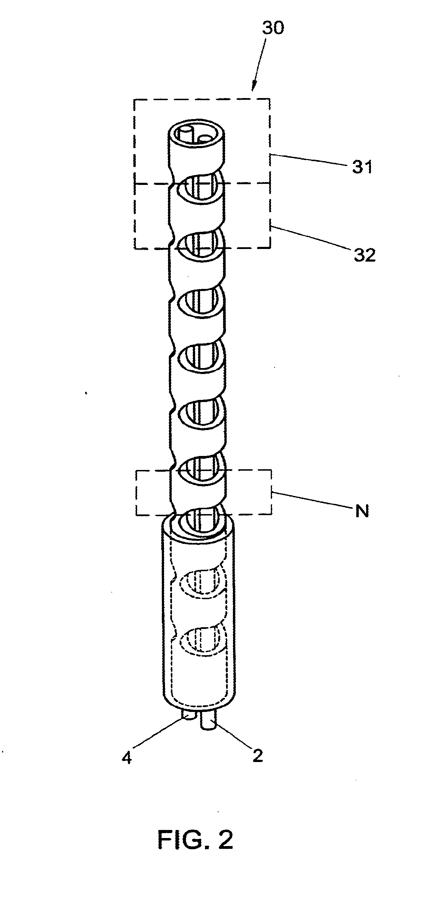

This technical feature is based on the insight that by providing a continuous structure having a plurality of interconnected bridge structures the accuracy of the bending is improved. In particular, when the elastic hinge comprises a plurality of interconnected hinge elements having substantially tubular cross-section and provided with recesses, controlled bending with preservation of the shape of the cross-section may be achieved. This is of particular advantage for medical applications. More details on this embodiment will be presented with reference to Figure 2.

These and other aspects of the invention will be further discussed with reference to drawings, wherein like reference signs represent like elements. It will be appreciated that the drawings are used for illustrative purposes only and may not be construed for limiting the scope of the appended claims.

BRIEF DESCRIPTION OF THE DRAWINGS

Figure 1 presents a schematic view of a bridge principle used in the bendable structure according to the invention.
Figure 2 presents a schematic view of a hinge comprising a plurality of hinge elements.
Figure 3 presents a schematic view of a medical device comprising the bendable structure according to the invention.

DETAILED DESCRIPTION OF THE DRAWINGS

Figure 1 presents a schematic view of a bridge principle used in the bendable structure according to the invention. The bendable structure 10 comprises a body 1, preferably a tubular body being manufactured from an elastic material. The body is provided with actuator comprising a first uni-directional SMA wire 4, a second uni-directional SMA wire 2 and a substantially rigid hinge 3 arranged to mechanically interconnect the first wire 2 and the second wire 4 for obtaining a bridge. An equivalent mechanical scheme is presented by element 20, illustrating the first wire 24, the second wire 22 and the bridging element 23. The first wire 4 is preferably pre-deformed by at least 4 - 8 % of its length at rest, i.e. before the bendable structure is operable. It is possible that the wire is elongated by at least 4 - 8 % of its length at rest. Due to the fact that the first wire is at least partially manufactured from a suitable uni-directional SMA material, in operation, upon heating above its phase transition temperature, for example by application of a current pulse of a suitable duration and amplitude, it will recover its normal shape, i.e. shorten thereby pulling the portion 3a of the hinge to the left. Due to the fact that the hinge 3 is manufactured from a substantially rigid material, the force applied by the first wire 4 to the portion 3a will be transferred substantially without loss to the portion 3b of the hinge 3. As a result the wire 2 will be elongated until equilibrium of forces is reached. As a result the second SMA wire 2 will store mechanical energy released by the first uni-directions SMA wired during relaxation. When the second wire is heated above its phase transition temperature, the second wire will resume its original length by pulling the portion 3b to the right. As a consequence the first SMA wire 4 will be elongated back to its original deformation condition. Thus, by alternating application of current pulses of suitable amplitude and duration, controlled bending of the body is reached. It will be appreciated that in order to bend the body in one direction at least two SMA wires 2, 4 are required. The hinge is connected to the body 1, which is schematically indicated by arms A, B. It will be appreciated that in practice instead of arms other technical means may be used, like adhesive or the like. Alternatively, the actuator comprising the hinge and the wires may be tightly fitted in the interior of the body 1 so that bending of the actuator is substantially transferred into bending of the body 1. It will be appreciated that for enabling controlled bending of the structure 10 in other directions additional wires may be required.

The hinge element 3 may be preferably manufactured from a tubular structure by means of laser ablation for producing voids having radius R. The absolute dimensions of the hinge 3 and the radius R depend on application. For example, for application in a bendable endoscope, it is possible that the outer dimension of the body is 0.7 mm, the inner dimension of the body is 0.5 mm L1 is about 0.5 mm, L2 is about 0.1 mm and radius R is about 0.5 mm. In order to determine feasible bending angles for this structure, the following equations are to be considered:

$$M = F * L \, ,$$

wherein

M represents moment applied to a point;
F represents force acting on a point;
L represents arm length.

$$F \doteq A * E * \varepsilon \quad \text{(Hook's law)}$$

During actuation of the perforated hinge as shown in Figure 2, the hinge will rotate in direction of the wire which is being actuated. Although the explanations are given with respect to the wires which are elongated and which shorten under application of the actuation pulse, it is also possible that the wire is shortened a-priori and elongates during application of the actuation pulse.

The rotation of the hinge proceeds until equilibrium of forces across the bridge (see Figure 1 element 20) is reached. In this state a sum of momenta in the bendable structure is zero, which is given by:

$$\sum M = 0$$

By filling in data pertaining to the geometry of the hinge, one obtains:

$$F1 * l1 - F2 * l2 - T\psi = 0$$

When the above equation is resolved with respect to the angle $\Psi$, one obtains that the forces equilibrium is maintained for the angle $\Psi$ of 7,2 degrees. By providing a suitable plurality of the hinge elements 31, 32, ..., N, as shown in Figure 2 a suitable bending angle is obtained. For example, for 30 elements forming the hinge 30 the bending angle of 216 degrees is reached. It is noted that when the body comprises an elastic hinge and the bridge structure is formed by the elastic hinge connected to the wire, in particular, when the elastic hinge is manufactured from a uni-directional SMA material, it is sufficient to use a sole actuating uni-directional SMA per desired bending direction, the elastic hinge acting as the second uni-directional SMA wire.

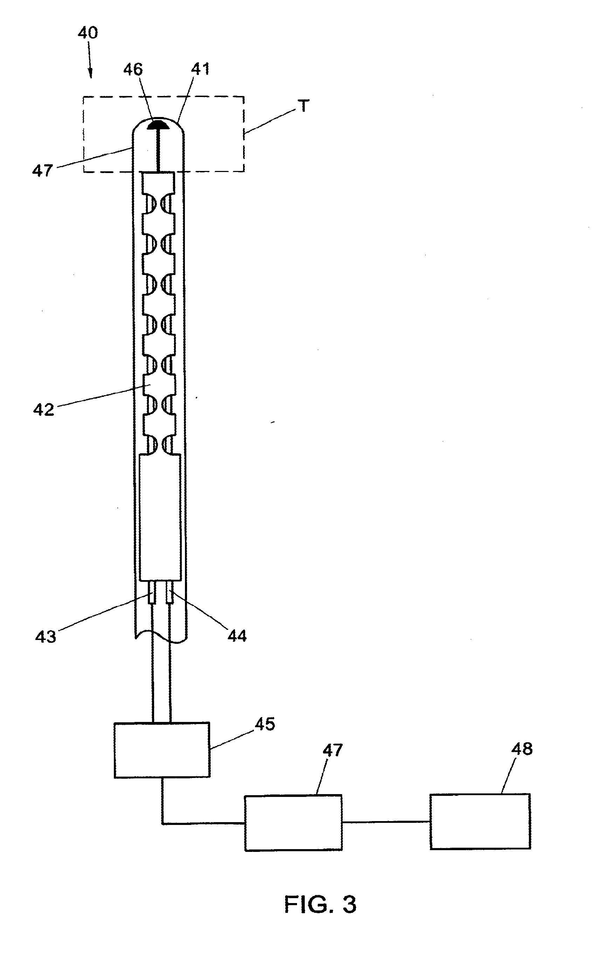

Figure 3 presents a schematic view of a medical device comprising the bendable structure according to the invention. The bendable structure 40 may relate to a bendable catheter or an endoscope. The catheter may be suitable to be maneuvered in a body's conduit, for example in a blood vessel or in a urinary tract. Below, example of the bendable structure implemented in a miniature endoscope will be given. It will be appreciated that a skilled artisan can employ the same teaching in application to catheters or any other suitable equipment requiring remote controlled bending.

Endoscope 40 may comprise an outer tubular body 41, whereto a flexible hinge 42 arranged with uni-directional SMA wires 43, 44 is attached. It is also possible that the endoscope 40 comprises a suitable lumen occupying only a portion of the internal volume, the hinge 42 together with the wires 43, 44, being arranged in the lumen. Preferably, the tubular body 41 has a diameter in the range of 0.5 - 10 mm, preferably in the range of 0.5 - 2 mm.

The uni-directional SMA wires may be actuated by means of application of a pulse of electrical current for heating the uni-directional SMA wire above its phase transition temperature. It is noted that either a transition of a suitable SMA material from martensitic phase to austenitic phase or from austenitic phase to R-phase is envisaged. In order to actuate the wires 43, 44 the endoscope 45 comprises a control unit, which is arranged to deliver the current pulse with required characteristics to the first wire 43 or to the second wire 45.

In a medical field, it is advantageous that a protrusion of the endoscope 40 in the human or animal body is being monitored in real-time. Preferably, such monitoring is arranged to prove a three-dimensional tracking of a position of the endoscope. Advantageously, the control unit 45 is arranged to receive the positional information on the endoscope together with imaging data on the area wherein the endoscope, in particular its tip portion T is dwelling. The bending angle can also be monitored using a strain sensor or optical fiber.

In accordance with the imaging data the control unit 45 or a suitable data analysis unit 47 arranged in communication with a suitable imaging unit 48 calculates the angle with which the tip portion T of the endoscope is to be bent prior to further insertion. It will be appreciated that the same procedure can be followed when an inspection endoscopy is performed, for example when the endoscope is positioned within an organ, or a cavity and is moved around to enable a wide view for the optical means 46. The optical means 46 is arranged in connection with an external device (not shown) for further processing of the data. Such connection is preferably implemented using optical fiber.

Preferably, the uni-directional SMA wires 43, 44 are arranged with a flattened cross-section. This has an advantage that less space is occupied by the wires 43, 44. This feature is of particular advantage for catheters conceived to be used in cardiac arteries, because miniaturization of cardiac devices plays a crucial role with respect to induced ischemia.

It will be appreciated that while specific embodiments of the invention have been described above, that the invention may be practiced otherwise than as described. In addition, isolated features discussed with reference to different figures may be combined. Although the bendable structure is explained with reference to endoscope other applications are contemplated, including other optical devices, for example for industrial application, catheters, or the like.

## Claims

1. A bendable structure (10) comprising:

   - a body (1) conceived to be bent;
   - an actuator for inducing a bending force in the body (1), wherein the actuator comprises
   - a first wire (4) manufactured from a uni-directional shape memory alloy (SMA) material and arranged in contact with a portion of the body (1) for forming a bridge structure; **characterized in that**, the first wire (4) is pre-deformed; and by further comprising:
   - a second deformable uni-directional SMA wire (2) joined by a rigid or flexible hinge (3) to the first pre-deformed uni-directional SMA wire (4), wherein, for enabling bending of the bendable structure (10), the second wire (2) is adapted to elongate in response to shortening of the first wire (4) by transfer of mechanical energy and the first wire (4) is adapted to elongate in response to shortening of the second wire(2) by transfer of mechanical energy.

2. A bendable structure (10) according to claim 1, wherein the body (1) comprises an elastic hinge (3), the bridge structure being formed by the elastic hinge (3) connected to the first and second wires.

3. A bendable structure (10) according to claim 2, wherein the body (1) comprises a plurality of interconnected hinges (3) extending in a longitudinal direction of the body (1).

4. A bendable structure (10) according to claim 2 or 3, wherein the body (1) comprises SMA material.

5. A bendable structure (10) according to claim 1, wherein the hinge (3) is a rigid hinge (3).

6. A bendable structure (10) according to claim 1, wherein the uni-directional SMA material is selected from a group of materials consisting of: NiTi, CuZnAl or CuAlNi.

7. A bendable structure (10) according to any preceding claim, wherein the body (1) has a low bending stiffness.

8. A bendable structure (10) according to any preceding claim, further comprising control means (45) for inducing a transition of the shape memory alloy from martensitic phase to austenitic phase or from austenitic phase to R-phase.

9. A bendable structure (10) according to claim 8, wherein the control means (45) is arranged for controlling of the uni-directional shape memory alloy by application of a current pulse of a short duration.

10. A bendable structure (10) according to any preceding claim, wherein the bridge structure is formed as a carrier of the body (1).

11. A bendable structure (10) according to any preceding claim, wherein the body (1) is tubular, having a diameter in the range of 0.5 - 10 mm, preferably in the range of 0.5 - 2 mm.

12. A bendable structure (10) according to any preceding claim, wherein the body (1) forms part of an optical device.

13. A bendable structure (10) according to claim 12, wherein the optical device is an endoscope (40).

14. A bendable structure (10) according to any preceding claim 1 - 11, wherein the body (1) forms part of a catheter.

15. A bendable structure (10) according to any preceding claim, wherein the first wire (4) and the second wire (2) have a flattened cross-section.

16. A bendable structure (10) according to any preceding claim, wherein the first wire is pre-deformed by elongation by 4 - 8 % of its initial length.

17. A bendable structure (10) according to any preceding claim 6 - 16, wherein the first wire and the second wire are pre-deformed.

## Patentansprüche

1. Biegbare Struktur (10), umfassend:

   - einen Körper (1), der konzipiert ist, um gebogen zu werden;
   - ein Betätigungselement zum Herbeiführen einer Biegekraft in dem Körper (1), wobei das Betätigungselement Folgendes umfasst:
   - einen ersten Draht (4), hergestellt aus einem unidirektionalen Formgedächtnislegierungsmaterial (SMA, engl. Shape Memory Alloy) und angeordnet in Kontakt mit einem Teil des Körpers (1), um eine Brückenstruktur zu formen, **dadurch gekennzeichnet, dass** der erste Draht (4) vorverformt ist; und ferner umfassend:
   - einen zweiten verformbaren unidirektionalen SMA-Draht (2), verbunden durch ein starres oder flexibles Gelenk (3) mit dem ersten vorverformten unidirektionalen SMA-Draht (4), wobei, um das Biegen der biegbaren Struktur (10) zu ermöglichen, der zweite Draht (2) geeignet ist, sich in Reaktion auf ein Verkürzen des ersten Drahtes (4) durch Übertragung von mechanischer Energie zu verlängern, und der erste Draht (4) geeignet ist, sich in Reaktion auf ein Verkürzen des zweiten Drahtes (2) durch Übertragung von mechanischer Energie zu verlängern.

2. Biegbare Struktur (10) nach Anspruch 1, wobei der Körper (1) ein elastisches Gelenk (3) umfasst, wobei die Brückenstruktur von dem mit dem ersten und dem zweiten Draht verbundenen elastischen Gelenk (3) geformt wird.

3. Biegbare Struktur (10) nach Anspruch 2, wobei der Körper (1) mehrere miteinander verbundene Gelenke (3) umfasst, die in einer Längsrichtung des Körpers (1) verlaufen.

4. Biegbare Struktur (10) nach Anspruch 2 oder 3, wobei der Körper (1) SMA-Material umfasst.

5. Biegbare Struktur (10) nach Anspruch 1, wobei das Gelenk (3) ein starres Gelenk (3) ist.

6. Biegbare Struktur (10) nach Anspruch 1, wobei das unidirektionale SMA-Material ein Material ist, ausgewählt aus einer Gruppe von Materialien, bestehend aus: NiTi, CuZnAl oder CuAlNi.

7. Biegbare Struktur (10) nach einem der vorhergehenden Ansprüche, wobei der Körper (1) eine geringe Biegesteifigkeit hat.

8. Biegbare Struktur (10) nach einem der vorhergehenden Ansprüche, ferner umfassend ein Kontrollmittel (45) zum Herbeiführen eines Übergangs der Formgedächtnislegierung aus einer martensitischen Phase in eine austenitische Phase oder aus einer austenitischen Phase in eine R-Phase.

9. Biegbare Struktur (10) nach Anspruch 8, wobei das Kontrollmittel (45) geeignet ist, die unidirektionale Formgedächtnislegierung durch Anwendung eines Stromimpulses von kurzer Dauer zu kontrollieren.

10. Biegbare Struktur (10) nach einem der vorhergehenden Ansprüche, wobei die Brückenstruktur als ein Träger des Körpers (1) geformt ist.

11. Biegbare Struktur (10) nach einem der vorhergehenden Ansprüche, wobei der Körper (1) röhrenförmig ist, mit einem Durchmesser im Bereich von 0,5 - 10 mm, bevorzugt im Bereich von 0,5 - 2 mm.

12. Biegbare Struktur (10) nach einem der vorhergehenden Ansprüche, wobei der Körper (1) einen Teil einer optischen Vorrichtung bildet.

13. Biegbare Struktur (10) nach Anspruch 12, wobei die optische Vorrichtung ein Endoskop (40) ist.

14. Biegbare Struktur (10) nach einem der vorhergehenden Ansprüche 1 - 11, wobei der Körper (1) Teil eines Katheters bildet.

15. Biegbare Struktur (10) nach einem der vorhergehenden Ansprüche, wobei der erste Draht (4) und der zweite Draht (2) einen abgeflachten Querschnitt haben.

16. Biegbare Struktur (10) nach einem der vorhergehenden Ansprüche, wobei der erste Draht durch Verlängerung um 4 - 8 % seiner ursprünglichen Länge vorverformt ist.

17. Biegbare Struktur (10) nach einem der vorhergehenden Ansprüche 6 - 16, wobei der erste Draht und der zweite Draht vorverformt sind.

**Revendications**

1. Structure pouvant être incurvée (10) comprenant :

   - un corps (1) conçu pour être incurvé ;
   - un actionneur pour induire une force d'incurvation dans le corps (1), dans lequel l'actionneur comprend :
   - un premier câble (4) fabriqué à partir d'un matériau en alliage à mémoire de forme (SMA) unidirectionnel et agencé en contact avec une partie du corps (1) pour former une structure de pont ;
   **caractérisée en ce que**, le premier câble (4) est pré-déformé ; et **en ce qu'**il comprend en outre :
   - un second câble SMA unidirectionnel déformable (2) relié par une articulation rigide ou souple (3) au premier câble SMA unidirectionnel prédéformé (4), dans lequel, pour permettre une incurvation de la structure pouvant être incurvée (10), le second câble (2) est adapté pour s'allonger en réponse à un raccourcissement du premier câble (4) par transfert d'énergie mécanique et le premier câble (4) est adapté pour s'allonger en réponse à un raccourcissement du second câble (2) par transfert d'énergie mécanique.

2. Structure pouvant être incurvée (10) selon la revendication 1, dans laquelle le corps (1) comprend une articulation élastique (3), la structure de pont étant formée par l'articulation élastique (3) reliée au premier et au second câble.

3. Structure pouvant être incurvée (10) selon la revendication 2, dans laquelle le corps (1) comprend plusieurs articulations reliées mutuellement (3) s'étendant dans une direction longitudinale du corps (1).

4. Structure pouvant être incurvée (10), selon les revendications 2 ou 3, dans laquelle le corps (1) comprend un matériau SMA.

5. Structure pouvant être incurvée (10) selon la revendication 1, dans laquelle l'articulation (3) est une articulation rigide (3).

6. Structure pouvant être incurvée (10) selon la revendication 1, dans laquelle le matériau SMA unidirectionnel est sélectionné parmi un groupe de matériaux constitué de : NiTi, CuZnAl ou CuAlNi.

7. Structure pouvant être incurvée (10) selon l'une quelconque des revendications précédentes, dans laquelle le corps (1) a une faible rigidité en incurvation.

8. Structure pouvant être incurvée (10) selon l'une quelconque des revendications précédentes, comprenant de plus des moyens de commande (45) pour induire une transition de l'alliage à mémoire de forme depuis une phase martensitique jusqu'à une phase austénitique ou depuis une phase austénitique jusqu'à une phase R.

9. Structure pouvant être incurvée (10) selon la revendication 8, dans laquelle les moyens de commande (45) sont agencés pour commander l'alliage à mémoire de forme unidirectionnel par application d'une impulsion de courant d'une courte durée.

10. Structure pouvant être incurvée (10) selon l'une quelconque des revendications précédentes, dans laquelle la structure de pont est formée en tant que support du corps (1).

11. Structure pouvant être incurvée (10) selon l'une quelconque des revendications précédentes, dans laquelle le corps (1) est tubulaire, ayant un diamètre dans la plage de 0,5 à 10 mm, de préférence dans la plage allant de 0,5 à 2 mm.

12. Structure pouvant être incurvée (10) selon l'une quelconque des revendications précédentes, dans laquelle le corps (1) forme une partie d'un dispositif optique.

13. Structure pouvant être incurvée (10) selon la revendication 12, dans laquelle le dispositif optique est un endoscope (40).

14. Structure pouvant être incurvée (10) selon l'une quelconque des revendications 1 à 11 qui précèdent, dans laquelle le corps (1) forme une partie d'un cathéter.

15. Structure pouvant être incurvée (10) selon l'une quelconque des revendications précédentes, dans laquelle le premier câble (4) et le second câble (2) ont une section transversale aplatie.

16. Structure pouvant être incurvée (10) selon l'une quelconque des revendications précédentes, dans laquelle le premier câble est pré-déformé par allongement de 4 à 8 % de sa longueur initiale.

17. Structure pouvant être incurvée (10) selon l'une quelconque des revendications 6 à 16 qui précèdent, dans laquelle le premier câble et le second câble sont pré-déformés.

FIG. 1

**FIG. 2**

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5679216 A